# EUROPÄISCHE PATENTSCHRIFT

(11) **EP 2 689 830 B1**
(45) Veröffentlichungstag und Bekanntmachung des Hinweises auf die Patenterteilung: **20.04.2016**
(21) Anmeldenummer: 12005444.0
(22) Anmeldetag: 26.07.2012
(51) Int. Cl.: B01F 7/00

(54) **Biogasanlage**
Bio gas facility
Installation de biogaz

(43) Veröffentlichungstag der Anmeldung: 29.01.2014
(73) Patentinhaber: Niederbacher, Michael, 39031 Bruneck (IT)
(72) Erfinder: Niederbacher, Michael, 39031 Bruneck (IT)
(74) Vertreter: Liebl, Thomas

(56) Entgegenhaltungen:
- EP-A2- 0 894 525
- AU-B3- 622 391
- DE-A1- 3 738 390
- DE-C1- 19 714 342
- DE-U1- 7 934 124
- DE-U1- 29 502 974
- DE-U1-202009 005 024
- US-A- 4 559 840
- US-A- 4 581 182

## Beschreibung

Die Erfindung betrifft eine Biogasanlage nach dem Oberbegriff des Anspruchs 1.

Biogasanlagen mit einem Fermenterbehälter, in dem ein Aggregatträger, vorzugsweise vertikal ausgerichtet, gelagert ist, sind allgemein bekannt. An dem Aggregatträger ist regelmäßig ein Aggregat, insbesondere ein Tauchmotorrührgerät, mittels einer Höhenverstelleinrichtung höhenverstellbar gehaltert.

Aus der EP 0 894 525 B1 ist bereits eine Höhenverstelleinrichtung für ein Tauchmotorrührgerät bekannt, wobei die Höhenverstelleinrichtung hier als Seilwinde ausgebildet ist, die eine mit dem Aggregatträger fest verbundene Seiltrommel mit horizontaler Seiltrommelwelle aufweist. Weiter ist ein einerseits mit dem Aggregat verbundenes und andererseits auf die Seiltrommel aufwickelbares Seil vorgesehen, wobei die Seiltrommel innerhalb des Fermenterbehälters angeordnet ist, so dass keine gasdichte Seildurchführung durch eine Behälterwand erforderlich ist. Der Seiltrommel ist ein Seiltrommelantrieb zugeordnet, die durch eine außerhalb des Behälters angeordnete Betätigungseinrichtung betätigbar ist.

Eine derartige Seilwinde als Höhenverstelleinrichtung weist eine Reihe von Nachteilen auf: So zum einen den Nachteil, dass das Seil in einem Fermenterbehälter geführt und aufgenommen ist, der zu fermentierende Stoffe bzw. Flüssigkeiten sowie Gase enthält, die für das regelmäßig als Stahlseil ausgebildete Seil ein stark korrosives Medium darstellen. Die Korrosionsgefahr wird zudem noch dadurch erhöht, dass bei auf die Seiltrommel aufgewickeltem Seil mehrere Seillagen dicht aneinander gepresst und kraftbeaufschlagt durch das am Seil hängende Aggregat aufgewickelt sind, was zum einen zu einer starken mechanischen Seilbeanspruchung, zum Beispiel zu Seileinrissen durch Scheuem etc, führt und zudem auch die Korrosionsgefahr für die einzelnen aneinanderliegenden Seilbereiche wesentlich erhöht. Diese im Laufe der Zeit fortschreitende mechanische Beanspruchung und Korrosion des Seils führt letztendlich dazu, dass es regelmäßig zu Seilbrüchen kommt, wodurch dann das daran gehaltene Aggregat in unerwünschter Weise nach unten auf den Boden des Fermenterbehälters absackt. Dies hat letztendlich zur Folge, dass der Betrieb des Fermenterbehälters und damit der gesamten Biogasanlage gestoppt werden muss und das Aggregat auf aufwändige Weise geborgen werden muss. Dies kann zum Beispiel durch den Einsatz von Tauchern erfolgen, was aber sicherheitstechnisch äußerst bedenklich ist. In der Praxis wird daher in einem solchen Schadensfall normalerweise der Fermenterbehälter entleert, anschließend geputzt, weil sich Sedimente oder Sinkschichten am Fermenterboden ablagern. Vor diesen Arbeiten muss der Behälter zudem belüftet werden, um das immer noch produzierte Biogas zu entfernen, damit dieses die Arbeiter nicht beeinträchtigt. Es ist daher in einem solchen Schadensfall mit einem erheblichen Produktionsausfall zu rechnen, da nach Behebung des Schadens die gesamte Biologie wieder neu aufgebaut und gestartet werden muss. Dies bedeutet im Schadensfall regelmäßig einen Stillstand der Anlage von zwei oder drei Monaten, was für eine Biogasanlage mitunter auch das finanzielle Aus bedeuten kann.

Weiter hat das Seil den Nachteil, dass es aufgewickelt werden muss und deshalb bei großen Seillängen auch eine relativ große Seiltrommel benötigt wird, die regelmäßig nicht durch Standard-Serviceöffnungen bzw. Standard-Serviceschächte passt.

Beim Seil werden zudem Schmutzstoffe, wie zum Beispiel langfaseriges Material etc. mit hochgezogen und wickeln sich um die Seiltrommel herum. Dies führt dann zu Verstopfungen und gegebenenfalls sogar zum Verlust der Höhenverstellung.

Ein weiterer wesentlicher Nachteil einer derartigen Seilwinde als Höhenverstelleinrichtung ist, dass das Seil ein nicht formstabiles, biegeschlaffes Bauteil ist, das nicht in der Lage ist, die gewünschte Führungsstabilität beim Absenken und Anheben des Aggregates zur Verfügung zu stellen, wodurch es insbesondere beim Durchstoßen der Schwimmschichten bzw. Schaumschichten des Fermenterbehälters regelmäßig zu Absenkproblemen kommt, da diese Schwimm- bzw. Schaumschichten oftmals so dicht sind, dass die Gefahr besteht, dass das Aggregat selbst in einem gewissen Maße "aufschwimmt" und dadurch nicht in der gewünschten Weise schnell und funktionssicher abgesenkt werden kann. Dadurch dass das Seil relativ "leicht" ist, besteht zudem die Gefahr, dass es vom Flügel durch den Sog, der hinter dem Rührwerk entsteht, angesaugt wird. Das Seil läuft damit Gefahr, bei einem unkontrolliertem Absenken, bei dem das Aggregat zum Beispiel in einer Schwimmschicht stecken bleibt, angesaugt zu werden und in den Flügel zu geraten. Das hat zur Folge, dass nicht nur das Seil reißen kann (was zu den bereits zuvor beschriebenen Nachteilen führt), sondern dass auch das Aggregat selbst beschädigt werden kann, indem zum Beispiel der oder die Flügel vorne abreißen; oder aber das Aggregat zieht sich nach vorne oben und schlägt gegen den eigenen Mast.

Falls das Aggregat abreißt und nach unten absackt bzw. abfällt, besteht zudem die Gefahr, dass es in evt. vorhandene Heizungsrohre gelangt oder gegen die Mauer und/oder andere Einbauteile schlägt.

Zudem erlaubt ein derartiges biegeschlaffes Seil keine vollständige Durchmischung des Behälters.

Ersichtlich stellt daher die Ausbildung einer Höhenverstelleinrichtung als Seilwinde mit mechanisch hoch beanspruchten Seillagen, bei der die Seiltrommel und das darauf aufwickelbare Seil zudem im Fermenterbehälterinnenraum angeordnet und damit dauerhaft einem korrosiven Medium bzw. einer korrosiven Atmosphäre ausgesetzt ist, ein erhebliches Gefahrenpotential für einen dauerhaften funktionssicheren Betrieb der Höhenverstelleinrichtung und damit des Fermenterbehälters und damit wiederum der gesamten Biogasanlage dar. Dieser Gefahr kann nur dadurch begegnet werden, dass das Zugseil im Rahmen von Servicearbeiten regelmäßig ausgetauscht wird, was für den Biogasanlagenbetreiber ersichtlich äußerst aufwändig ist.

Des Weiteren ist aus der DE 20 2009 005 024 U1 eine Höhenverstellvorrichtung für ein Tauchrühr- oder Pumpwerk einer Biogasanlage bekannt, wobei das Rührwerk oder Pumpwerk mittels eines Höhenverstellmittels in der Höhe verstellbar ist. Das Höhenverstellmittel ist durch einen Hydraulikzylinder und ein außerhalb des Gärbehälters angeordnetes, einen Hydraulikdruck erzeugendes Mittel gebildet, wobei ein befestigtes Ende des Hydraulikzylinders in einer ortsfesten Aufnahme angeordnet ist und ein distales Ende über eine Seilverbindung mit dem Rührwerk verbunden ist. Außerhalb des Fermenterbehälters ist eine durch voneinander beabstandete Rollen gebildete Umlenkvorrichtung für das Seil angeordnet. Der Abstand zwischen den Rollen kann durch die Hydraulikzylindereinheit verstellt werden. DE 20 2009 005 024 U1 offenbart den Oberbegriff des Anspruchs 1.

Ein höhenverstellbar an einer vertikalen Stützstange gehaltertes Tauchmotorrührgerät ist auch aus der US 4,581,182 bekannt. Konkret wird hierbei das Tauchmotorrührgerät in der Höhe mittels einer Seilwinde verstellt, wobei das Seil an einem Kragarm festgelegt und über Umlenkrollen zu der außerhalb des Behälters angeordneten Seilwinde geführt ist.

Die DE 295 02 974 U1 betrifft eine Rühreinrichtung für einen Biogasbehälter mit einem Tauchrührwerk, das höhenverstellbar an einem vertikalen Mast geführt ist. Im oberen Bereich des Mastes ist außerhalb des Fermenterbehälters eine Seilwinde angebracht, deren Drahtseil von außerhalb des Behälters und damit von der Seilwinde kommend am Mast entlang bis in den flüssigkeitsbefüllten Siphon geführt ist, dort um das innere, mit der Wandung oder der Decke dicht verbundene Siphonrohr nach oben und schließlich um das äußere, mit dem Mast verbundene und nach unten abgeschlossene Siphonrohr nach unten zum Tauchrührwerk umgelenkt wird.

Und schließlich ist aus der DE 197 14 342 C1 eine Biogasanlage mit einer höhenverstellbaren Rühreinheit bekannt, bei der oberhalb einer Serviceöffnung ein Servicedom angeordnet ist, in den ein Tauchmotorrührwerk, höhenverstellbar geführt an einem vertikalen Mast, mittels einer Seilwinde bewegt werden kann. Das Seil der Seilwinde ist dabei im Inneren des Mastes geführt und über Umlenkrollen zum Tauchmotorrührwerk geführt.

Es ist daher Aufgabe der vorliegenden Erfindung, eine Biogasanlage mit einem Fermenterbehälter zur Verfügung zu stellen, mittels dem die Aggregatverstellung entlang eines Aggregatträgers im Fermenterbehälter auf einfache und funktionssichere Weise auch über einen längeren Zeitraum bewerkstelligt werden kann.

Diese Aufgabe wird gelöst mit den Merkmalen des Patentanspruchs 1. Vorteilhafte Ausgestaltungen hierzu sind Gegenstand der darauf rückbezogenen Unteransprüche.

Gemäß Patentanspruch 1 wird eine Biogasanlage mit einem Fermenterbehälter vorgeschlagen, in dem ein vertikal ausgerichteter Aggregatträger gelagert ist, wobei ein Aggregat, insbesondere ein Tauchmotorrührgerät, mittels einer Höhenverstelleinrichtung höhenverstellbar an dem Aggregatträger gehaltert ist. Die Höhenverstellvorrichtung ist durch einen Umschlingungstrieb gebildet, dessen Zugmittel um wenigstens zwei in Aggregatträger-Längsrichtung voneinander beabstandete Umlenkelemente geführt ist, wobei wenigstens eines der Umlenkelemente mittels einer Antriebseinrichtung drehantreibbar ist und wobei das Aggregat dergestalt mit dem Zugmittel gekoppelt ist, dass dieses bei einer Drehbetätigung des Umlenkelementes in eine erste Richtung angehoben und bei einer Drehbetätigung des Umlenkelementes in eine zweite, entgegengesetzte Richtung abgesenkt wird. Erfindungsgemäß wird vorgeschlagen, dass das Zugmittel von einem Anbindungspunkt am Aggregat ausgehend in Hochachsenrichtung nach oben um ein vorzugsweise drehantreibbares Umlenkelement und von dort ausgehend nach unten zu wenigstens einem unteren Umlenkelement geführt ist, von wo ausgehend das Zugmittel wieder nach oben zum Aggregat zurückgeführt und dort angebunden ist.

Die Anbindung des Zugmittels kann grundsätzlich am selben Anbindungspunkt am Aggregat erfolgen; besonders bevorzugt ist jedoch eine Anbindung der beiden freien Endbereiche des Zugmittels an voneinander beabstandeten aggregatsseitigen Anbindungspunkten, zum Beispiel in Verstellrichtung gesehen mehr oder weniger gegenüberliegenden aggregatseitigen Anbindungspunkten. Auch eine versetzte Anbindung ist zum Beispiel möglich.

Der besondere Vorteil dieser erfindungsgemäßen Lösung liegt darin, dass mit einem derartigen Umschlingungstrieb auf besonders einfache Weise sichergestellt werden kann, dass das Aggregat zum Beispiel beim Absenken durch die fermenterbehälterseitigen Schwimm- bzw. Schaumschichten hindurch gezogen werden kann, da mittels eines derartigen Umschlingungstriebes auf einfache Weise sichergestellt wird, dass beim Absenken eine entsprechend nach unten gerichtete Zugkraftkomponente auf das Aggregat wirkt. Zudem verhindert ein derartiger Umschlingungstrieb zuverlässig, dass das Zugmittel vom Flügel durch den Sog, der hinter einem Rührwerk oder dergleichem Aggregat entsteht, angesaugt werden kann.

Mit einem derartigen Aufbau lässt sich zudem auf vorteilhafte Weise die Bruchgefahr hinsichtlich des Zugmittels deutlich verringern, da ein derartig erfindungsgemäß eingesetztes Zugmittel, zum Beispiel in Form einer Zugkette, ausreichend stabil ausgeführt werden kann, so dass das Zugmittel selbst im leicht korrodierten Zustand noch eine solche ausreichende Festigkeit aufweist, um ein Aggregat im Medium des Fermenterbehälters funktionssicher und zuverlässig anheben und absenken zu können.

Des Weiteren braucht ein derartiges Zugmittel nicht auf eine Seiltrommel aufgewickelt zu werden, so dass insgesamt eine kompakte Bauweise vorliegt und das Zugmittel keine kraftbeaufschlagten und damit eng zusammen- und aneinandergepressten, mechanisch hoch beanspruchten Bereiche aufweist. Zudem können aufgrund der fehlenden Aufwicklung des Zugmittels auf eine Seiltrommel auf einfache Weise auch sehr große Höhenverstellungen eines Aggregates, von zum Beispiel weit über 6m, realisiert werden.

Das Zugmittel kann grundsätzlich auf unterschiedliche Weise ausgebildet sein, so zum Beispiel in der Art eines Profilriemens oder Zahnriemens, gegebenenfalls sogar durch ein Seil (zum Beispiel in Verbindung mit kurzen Wartungs- und Intervall-bzw. Austauschzeiten des Seils). Besonders bevorzugt ist das Zugmittel jedoch durch eine stabile Zugkette gebildet, vorzugsweise durch eine mehrdimensional bewegliche Kette, insbesondere eine Gliederkette bzw. Rundgliederkette, oder alternativ durch eine lediglich eindimensional bewegliche Gelenkkette. Eine derartige lediglich eindimensional bewegliche Gelenkkette kann wiederum durch eine Rollenkette oder eine Buchsenkette oder eine Bolzenkette oder dergleichen Gelenkketten gebildet sein. Im Falle einer Zugkette ist dann das drehantreibbare Umlenkelement bevorzugt durch wenigstens ein drehbar gelagertes Kettenrad, wie zum Beispiel eine Kettennuss, gebildet, das mit der Zugkette formschlüssig in Eingriff steht. Ein weiterer wesentlicher Vorteil einer derartigen Ausbildung des Zugmittels als Zugkette, insbesondere als zum Beispiel in der Art einer Fahrradkette ausgebildete eindimensional bewegliche Gelenkkette ist, dass diese schwerer als ein Zugseil ist bzw. gegebenenfalls in definierten Richtungen formstabil ist und somit anders als ein biegeschlaffes Seil eine gewisse Führungsstabilität zur Verfügung stellt, die in Verbindung mit insbesondere dem Absenken eines Aggregats von Vorteil ist, da hierdurch die sich im Fermenterbehälter eventuell ausbildenden Schwimm- bzw. Schaumschichten einfacher durchdrungen werden können und ein Auswandern des Aggregates zuverlässig vermieden werden kann. Insgesamt verleiht daher eine derartige Zugkette, insbesondere in Form einer eindimensional beweglichen Gelenkkette, dem Aufbau insgesamt eine größere Stabilität.

Mit der erfindungsgemäßen Lösung wird somit ein gegenüber der EP 0 894 525 B1 vollkommen neues und auf einem anderen Funktions- und Wirkprinzip beruhendes Konzept einer Höhenverstelleinrichtung in Verbindung mit Fermenterbehältern von Biogasanlagen zur Verfügung gestellt.

An dieser Stelle sei ausdrücklich erwähnt, dass die Begrifflichkeit "Fermenterbehälter" als Oberbegriff für sämtliche Behälter einer Biogasanlage steht, in denen ein Aggregat höhenverstellbar gelagert sein kann; insofern ist der Begriff "Fermenterbehälter" hier weit und umfassend auszulegen.

Gemäß einer besonders bevorzugten konkreten Ausgestaltung der vorliegenden Erfindungsidee wird vorgeschlagen, dass die einzelnen Gelenkachsen der Gelenkkette im Wesentlichen achsparallel zur Drehachse des Kettenrades ausgebildet sind, so dass die Gelenkkette in der gewünschten Weise in den beiden anderen Richtungen eine gewisse Formstabilität und Steifigkeit aufweist, die dazu beiträgt, die Führungsstabilität der Höhenverstelleinrichtung beim Absenken und Anheben des Aggregats zu verbessern.

Bildet das obere, gut zugängliche Umlenkelement das drehangetriebene Umlenkelement aus, ist es somit auch für Service- und Reparaturarbeiten gut zugänglich. Das untere Umlenkelement kann mittelbar oder unmittelbar an dem Aggregatträger angeordnet sein und zum Beispiel auch durch eine Führungsschiene oder dergleichen gebildet sein. Besonders bevorzugt ist jedoch eine Ausgestaltung, bei der das untere Umlenkelement ebenfalls drehbar gelagert ist, zum Beispiel als Führungsrad ausgebildet ist. Ganz besonders bevorzugt ist eine Ausgestaltung, bei der für den Fall, dass das Zugmittel durch eine Zugkette gebildet ist, auch das untere Umlenkelement durch ein Kettenrad gebildet ist, das analog dem zuvor beschriebenen Kettenrad in Eingriff mit der Zugkette steht, wodurch auf einfache Weise sichergestellt wird, dass die Zugkette entlang des gesamten Umschlingungstriebs zuverlässig und funktionssicher geführt und formschlüssig gehalten ist. Insbesondere in Verbindung mit einem derartigen Umschlingungstrieb ist auch die Ausgestaltung des Zugmittels als eindimensional bewegliche Gelenkkette besonders vorteilhaft, da hierdurch auf einfache Weise sichergestellt wird, dass die Gelenkkette als Zugmittel in Gelenkachsenrichtung der Gelenkkette nicht ausknicken kann und somit auf einfache Weise sichergestellt wird, dass die Gelenkkette als Zugmittel in der vorgesehenen Umschlingungstriebebene gehalten wird.

Es versteht sich, dass der Umschlingungstrieb selbst auch noch weitere zusätzliche Umlenkelemente bzw. drehbare oder nicht-drehbare Umlenkelemente, wie zum Beispiel Kettenräder, aufweisen kann, was zum Beispiel auch von der Länge des Zugmittels abhängt. Mit derartigen zusätzlichen Umlenkelementen kann auf einfache Weise die Funktionssicherheit und Betriebssicherheit des Umschlingungstriebs gewährleistet werden. Zudem kann auch ein, zum Beispiel durch ein derartiges Umlenkelement gebildetes, Spannelement vorgesehen sein, mittels dem zum Beispiel die Kettenspannung einstellbar ist, was ebenfalls dazu beiträgt, die Betriebssicherheit des Umschlingungstriebs und damit der Höhenverstelleinrichtung zu gewährleisten.

Ein derartiger Umschlingungstrieb ist bevorzugt im Behälterinnenraum des Fermenterbehälters angeordnet, wobei das drehantreibbare Umlenkelement oder eine mit diesem wirkverbundene Antriebseinrichtung mittels einer Betätigungseinrichtung von außerhalb des Fermenterbehälters betätigbar ist. Eine derartige Anordnung im Behälterinnenraum umfasst ausdrücklich Behälterausgestaltungen, bei der die Anordnung der erfindungsgemäßen Höhenverstelleinrichtung in einem domartigen Bereich eines Serviceschachts erfolgt.

Die Umlenkelemente des Umschlingungstriebs sind beabstandet voneinander mittelbar oder unmittelbar am Aggregatträger festgelegt, zum Beispiel über Halte- und Montageplatten und- winkel, wodurch eine einfache Montage und Anordnung des Umschlingungstriebs sichergestellt ist.

Dem wenigstens einen Umlenkelement des Umschlingungstriebs kann wenigstens ein ein- oder mehrteiliges Führungs- und/oder Leitelement, zum Beispiel ein Leitblech oder dergleichen, dergestalt zugeordnet sein, dass das Zugmittel, insbesondere eine Zugkette, wie zum Beispiel eine Gliederkette oder eine Gelenkkette, dauerhaft in Eingriff mit dem zugeordneten Umlenkelement, vorzugsweise einem Kettenrad, gehalten ist. Hierzu ist das wenigstens eine, ein- oder mehrteilige Führungs- und/oder Leitelement bevorzugt mit einem definierten Spaltabstand über wenigstens einen Teilbereich des jeweiligen Umlenkelementes herumgeführt. Ein derartiges Führungs- und/oder Leitelement kann grundsätzlich auf unterschiedliche Weise ausgebildet sein. Zum Beispiel kann ein in Hochachsenrichtung oberes Führungs- und/oder Leitelement so ausgebildet sein, insbesondere so mehrteilig ausgebildet sein, dass dieses eine Austrittsöffnung oder einen Austrittspalt für hochgezogene Feststoffe ausbildet, über den die hochgezogenen Verunreinigungen, wie zum Beispiel Faserstoffe, aus dem Bereich des Umschlingungstriebes austreten und abgesondert werden können. Bevorzugt ist auf jeden Fall auch eine Ausgestaltung, bei der das Leitelement das Umlenkelement, zum Beispiel ein Kettenrad im Wesentlichen über einen Großteil oder auch den gesamten Umlenk- bzw. Eingriffsbereich überdeckt, um sicherzustellen, dass auch stets die zum Beispiel gewünschte Zähnezahl eines Kettenrades, um nur ein Beispiel zu nennen in Eingriff mit dem zugeordneten Eingriffsbereich der Zug- bzw. Gelenkkette steht.

Sämtlichen Ausführungsformen ist gemein, dass dem drehantreibbaren Umlenkelement eine vorzugsweise elektrisch, hydraulisch, pneumatisch oder manuell betreibbare Antriebsvorrichtung zugeordnet sein kann, mittels der das Umlenkelement auf einfache Weise mittelbar oder unmittelbar drehangetrieben werden kann. Gemäß einer hierzu besonders bevorzugten Ausführungsform ist vorgesehen, dass das Umlenkelement Bestandteil einer Antriebswelle ist, die ferner ein Antriebsritzel aufweist, das mit einem Getriebe zusammenwirkt, das das Antriebsritzel und damit das Umlenkelement antreibt. Ein derartiger Aufbau ermöglicht viele konstruktive Freiheiten und ist zudem sehr funktionssicher in der täglichen Praxis sowie in der Bedienung. Dies trifft vor allem auf den besonders bevorzugten Fall zu, dass das Getriebe Bestandteil einer Hohlwellenanordnung ist, bei der in einer, gegebenenfalls ein Deckenwand-Drehlager ausbildenden und/oder gegebenenfalls Bestandteil des Aggregatträgers bildenden, Hohlwelle eine Antriebswelle drehbar gelagert ist, die einen mit dem Antriebsritzel mittelbar oder unmittelbar kämmenden Eingriffsbereich aufweist. Zudem ist diese Antriebswelle bevorzugt mit einem freien Ende aus dem Fermenterbehälter herausgeführt. Vor allem letzteres ermöglicht auf besonders einfache und bedienungssichere Weise das Vorsehen eines die Antriebswelle antreibenden Antriebsmotors, der durch eine Betätigungseinrichtung betätigbar ist. Grundsätzlich kann ein derartiges Getriebe auf vielfache Weise ausgebildet sein. Besonders bevorzugt ist jedoch vorgesehen, dass das Getriebe ein Schneckentrieb mit einer Schneckenwelle als Antriebswelle ist, deren Eingriffsbereich durch ein Schneckengewinde gebildet ist. Ein derartiger Schneckentrieb ist besonders robust und weist eine vorteilhafte Selbsthemmung auf, die sicherstellt, dass eingestellte Höhenpositionen des Aggregates funktionssicher gehalten werden können.

Die Erfindung wird nachfolgend anhand einer Zeichnung näher erläutert.

Es zeigen:
- Fig. 1: eine schematische Teilansicht eines Fermenterbehälters einer Biogasanlage mit einem darin vertikal gelagerten Aggregatträger und einer beispielhaften Ausführungsform einer erfindungsgemäßen Höhenverstelleinrichtung,
- Fig. 2: schematisch eine vergrößerte Detaildarstellung des Umschlingungstriebs der Fig. 1,
- Fig. 3: schematisch eine Schnittansicht entlang der Y-Y der Fig. 2,
- Fig. 4a: schematisch eine beispielhafte Darstellung eines Kettenbereichs einer zum Beispiel als Rollenkette ausgebildeten Gelenkkette,
- Fig. 4b: eine um 90° gekippte Anordnung des Kettenbereichs der Fig. 4a,
- Fig. 5a: schematisch eine weitere beispielhafte und zur Fig. 1 alternative Ausführungsform mit einer Hohlwellenanordnung,
- Fig. 5b: eine schematische Schnitt- und Detailansicht durch das obere, als Hohlwellenanordnung ausgebildete Drehlager des Aggregatträgers gemäß Fig. 5a.

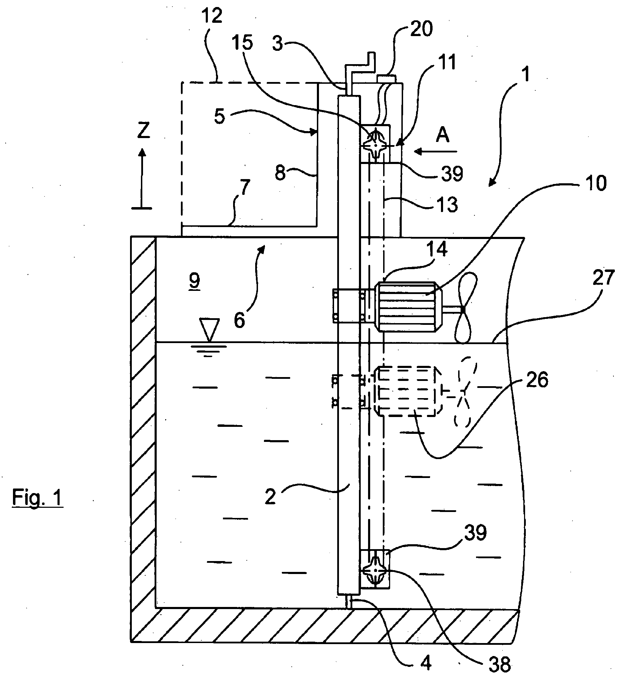

In der Fig. 1 ist beispielhaft und schematisch eine Teilansicht eines Fermenterbehälters 1 einer Biogasanlage gezeigt, in dem ein Aggregatträger 2 vertikal ausgerichtet gelagert ist.

Der Aggregatträger 2 kann grundsätzlich auf unterschiedliche Weise ausgebildet sein, zum Beispiel rund. In der hier gezeigten Ausführungsform ist der Aggregatträger 2 durch ein Quadratleitrohr mit quadratischem Querschnitt gebildet.

Der Aggregatträger 2 ist drehbar, das heißt um seine Längsachse drehbar im Fermenterbehälter 1 gelagert. Hierzu weist der Aggregatträger 2 ein unteres Drehlager 3 sowie ein oberes Drehlager 4 auf. Das untere Drehlager 3 ist durch die Fermenterbehälter-Bodenwand ausgebildet, während das obere Drehlager 4 hier beispielhaft durch eine Deckenwand eines L-Serviceschachtes 5 gebildet ist. Dieser L-Serviceschacht 5 überdeckt eine hier nicht im Detail dargestellte deckenwandseitige Serviceöffnung 6 des Fermenterbehälters 1, damit keine Gase aus dem Behälterinnenraum 9 in die Umgebung entweichen können. Der L-Serviceschacht 5 weist zum Beispiel zwei Wandplatten 7, 8 auf, die im Servicefall vom L-Serviceschacht 5 abgenommen werden können, so dass über die Serviceöffnung 6 ein Zugang in den Fermenterbehälterinnenraum 9 ermöglicht wird.

Im Servicefall kann dann das am Aggregatträger 2 höhenverstellbar gelagerte Aggregat 10, das hier beispielhaft durch ein Tauchmotorrührgerät gebildet ist, durch Verschwenken des Aggregatträgers 2 um dessen Längsachse aus der in der Bildebene der Fig. 1 gezeigten rechten Position um 180° nach links geschwenkt werden, und anschließend durch Betätigen der nachfolgend noch näher erläuterten Höhenverstelleinrichtung 11 so weit nach oben angehoben werden, dass das Aggregat 10 über die Serviceöffnung 6 zugänglich ist bzw. durch die Serviceöffnung 6 hindurch nach außerhalb des Fermenterbehälters 1 verlagert werden kann.

Anstelle eines L-Serviceschachts 5, wie er in der Fig. 1 gezeigt ist, kann auch ein kastenförmiger Serviceschacht 12 vorgesehen sein, wie er in der Fig. 1 lediglich strichliert eingezeichnet ist, in den das Aggregat 10 zu Service- und Reparaturarbeiten hinein bewegt werden kann und der zum Beispiel über eine Zugangstür oder Zugangsklappe zugänglich ist.

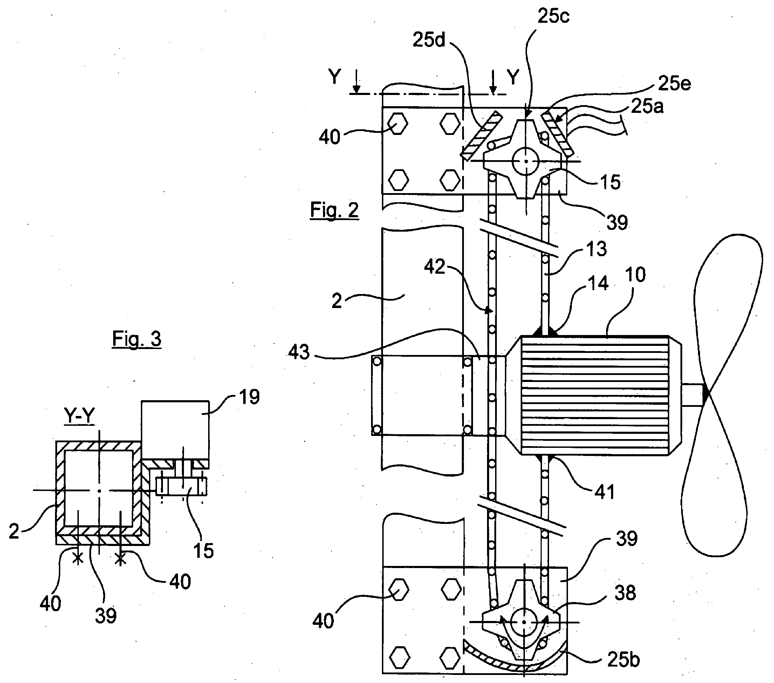

Die Höhenverstelleinrichtung 11 ist hier durch einen Umschlingungstrieb gebildet, bei dem eine hier beispielhaft lediglich eindimensional bewegliche Gelenkkette 13 als Zugmittel bzw. Zugkette von einem ersten Anbindungspunkt 14 am Aggregat 10 ausgehend in Hochachsenrichtung z nach oben um ein Kettenrad 15 und von dort ausgehend nach unten zu einem hier beispielhaft unteren Kettenrad 38 als unterem Umlenkelement geführt ist, das am Aggregatträger 2 drehbar gelagert ist. Wie dies insbesondere der Fig. 2 entnommen werden kann, können bei dieser Ausführungsform die beiden Kettenräder 15, 38 über Montageböcke oder -platten 39 zum Beispiel mittels mehrerer Schraubverbindungen 40 mittelbar oder wie hier gezeigt unmittelbar am Aggregatträger 2 befestigt werden. Hierzu können die Montageböcke bzw. -platten 39 bevorzugt so abgewinkelt ausgebildet sein, dass die Drehebenen der Kettenräder 15, 38 in etwa in der Längsmittenebene des Aggregatträgers 2 liegen, so dass das Aggregat 10 selbst in etwa mittig ausgerichtet entlang des Aggregatträgers 2 höhenverstellbar ist. Es versteht sich, dass selbstverständlich auch eine gegenüber der Längsmittenebene des Aggregatträgers 2 versetzte Anordnung des Aggregats 10 grundsätzlich möglich ist.

Vom unteren Kettenrad 38 ausgehend ist die Gelenkkette 13 dann wieder nach oben zum Aggregat 10 zurückgeführt und dort mit seinem zweiten freien Endbereich an einem zweiten Anbindungspunkt 41 angebunden, der hier lediglich beispielhaft beabstandet und unterhalb des ersten Anbindungspunktes 14 des ersten freien Gelenkkettenendbereichs liegt.

Mit einem derartigen Aufbau wird somit ein Umschlingungstrieb ausgebildet, in den das Aggregat 10 dergestalt eingekoppelt ist, dass das Aggregat 10 bei einer Drehbetätigung des Kettenrades 15 über einen beispielsweise an der oberen Montageplatte 19 und/oder am Aggregatträger 2 angeflanschten elektrischen, pneumatischen oder hydraulischen Antriebsmotor 19 in eine erste Richtung angehoben werden kann und bei einer Drehbetätigung des Kettenrades 15 in eine zweite, entgegengesetzte Richtung abgesenkt werden kann.

Der Antriebsmotor 19 ist hier über eine Verrohrung bzw. Verschlauchung oder Verkabelung 21 mit einer außerhalb des Fermenterbehälters 1 angeordneten Betätigungseinrichtung 20 gekoppelt bzw. wirkverbunden.

Wie dies insbesondere aus der Fig. 2 ersichtlich ist, ist der freie Kettenbereich 42 der Gelenkkette 13, das heißt derjenige Kettenteilbereich zwischen den beiden Kettenrädern 15 und 38, in den das Aggregat 10 nicht eingekoppelt ist, hier beispielhaft durch eine aggregatseitig ausgebildete Durchführung 43 behinderungsfrei hindurchgeführt. Diese aggregatseitige Durchführung 43 kann beispielsweise als Schlitz, Spalt oder dergleichen Ausnehmung, zudem mit oder ohne Führungselement, wie zum Beispiel eine Führungsrolle oder dergeichen, ausgebildet sein. Alternativ dazu könnten die Kettenräder 15, 38 aber auch so gegenüber der Längsmitte des Aggregatträgers 2 versetzt sein, dass, zum Beispiel bei entsprechend groß ausgebildeten Kettenraddurchmessern, die Freigängigkeit des freien Kettenbereichs 42 über die Anordnung und/oder Dimensionierung der Kettenräder 15, 38 sichergestellt wird. Weiter alternativ könnte gegebenenfalls die Anordnung der Kettenräder 15 und 38 auch so sein, dass der freie Kettenbereich 42 im Inneren des Aggregatträgers 2 verläuft, der vorzugsweise als Hohlprofilträger ausgebildet ist. Ferner könnten auch weitere Umlenkelemente bzw. Spannelemente vorgesehen sein, die zum Beispiel den hier gezeigten Umschlingungstrieb dergestalt ergänzen und erweitern, dass die Kettenführung flaschenzugartig ausgebildet ist und sichergestellt ist, dass der freie Kettenbereich 42 immer außerhalb des Bewegungsbereiches des Aggregates 10 liegt.

Mit einem Spannelement kann zudem die Kettenspannung in der gewünschten Weise aufrecht erhalten werden, um ein Herausspringen der Kette zu vermeiden. Alternativ bzw. auch zusätzlich hierzu kann in Verbindung mit beiden Kettenrädern 15, 38, wie in der Fig. 2 schematisch dargestellt, auch wiederum vorgesehen sein, dort Leitelemente 25 vorzusehen, die einen solchen Spaltabstand zum Kettenrad aufweisen, dass die Gelenkkette 13 dauerhaft in einer Eingriffsverbindung mit dem jeweils zugeordneten Kettenrad 15 bzw. 38 gehalten wird.

Soll nun das Aggregat 10, wie in der Fig. 1 schematisch dargestellt, aus der mit durchgezogenen Linien gezeigten angehobenen Position nach unten in den Fermenterbehälter 1 abgesenkt werden, wird über die Betätigungseinrichtung 20 der Antriebsmotor 19 für eine vorbestimmte Zeitdauer so angesteuert, dass dieser das Kettenrad 15 im Uhrzeigersinn solange verdreht, bis das Aggregat 10 in die gewünschte, hier beispielhaft mit strichlierten Linien dargestellte abgesenkte Position 26 abgesenkt ist. Ist die gewünschte Position erreicht, wird der Antriebsmotor 19 gestoppt und damit das Kettenrad 15 angehalten, wodurch das Aggregat 10 sicher in der gewünschten abgesenkten Position 26 gehalten wird.

Es versteht sich, dass die Länge der Gelenkkette 13 ebenso wie der Abstand der beiden Kettenräder 15, 38 selbstverständlich so vorgegeben ist, dass damit sämtliche gewünschten Höhenpositionen des Aggregats 10 im Fermenterbehälter 1 eingenommen werden können.

Soll das Aggregat 10 dann von der in der Fig. 1 strichliert dargestellten abgesenkten Position 26 wieder in die mit durchgezogenen Linien dargestellte höhere Position angehoben werden, wird wiederum über die Betätigungseinrichtung 20 der Antriebsmotor 19 angesteuert, und zwar diesmal so, dass das Kettenrad 15 im Gegenuhrzeigersinn gedreht wird, wodurch wiederum der am Aggregat 10 am ersten Anbindungspunkt 14 angebundene Bereich der Gelenkkette 13 und somit das Aggregat 10 nach oben gezogen wird.

Es versteht sich weiter, dass die erfindungsgemäße Lösung selbstverständlich mit jeder Art von Behältern bzw. Fermenterbehältern einsetzbar ist, das heißt insbesondere auch mit Fermenterbehältern mit einer festen, horizontalen Deckenwand als auch mit Fermenterbehältern mit einem Foliendach oder dergleichen.

In Hochachsenrichtung z oberhalb des oberen drehantreibbaren Kettenrades 15 und unterhalb des unteren Kettenrades 38 ist jeweils ein Leitelement 25a bzw. 25b vorgesehen, das einen definierten Spaltabstand zum jeweiligen Kettenrad 15, 38 aufweist, dergestalt, dass die Gelenkkette 13 dauerhaft in einer Eingriffsverbindung mit dem Kettenrad 15 gehalten ist. Das untere Leitelement 25b ist hier beispielhaft bogenförmig gekrümmt ausgebildet und zum Beispiel in Form eines Leitbleches oder dergleichen um einen Großteil des Eingriffsbereichs des unteren Kettenrades 38 herumgeführt. Das obere Leitelement 25a ist dagegen hier beispielhaft zweiteilig durch voneinander beabstandete und zwischen sich eine Austrittsöffnung 25c ausbildende, hier beispielhaft dachförmig schräg angestellte Leitbacken 25d, 25e gebildet, wodurch sichergestellt ist, dass der Gelenkkette 13 hochgezogene Feststoffe über die Austrittsöffnung 25c funktionssicher abgeführt werden können und damit eine Verstopfungsgefahr im Bereich des oberen Kettenrades 15 vermieden wird. Derartige Leitelemente 25a, 25 b stellen somit eine funktionssichere Eingriffsverbindung zwischen der Gelenkkette 13 und dem jeweiligen Kettenrad 15, 38 sicher und helfen somit insbesondere ein Herausspringen der Gelenkkette 13 aus der Eingriffsverbindung mit dem Kettenrad 15 bzw. 38 zu vermeiden.

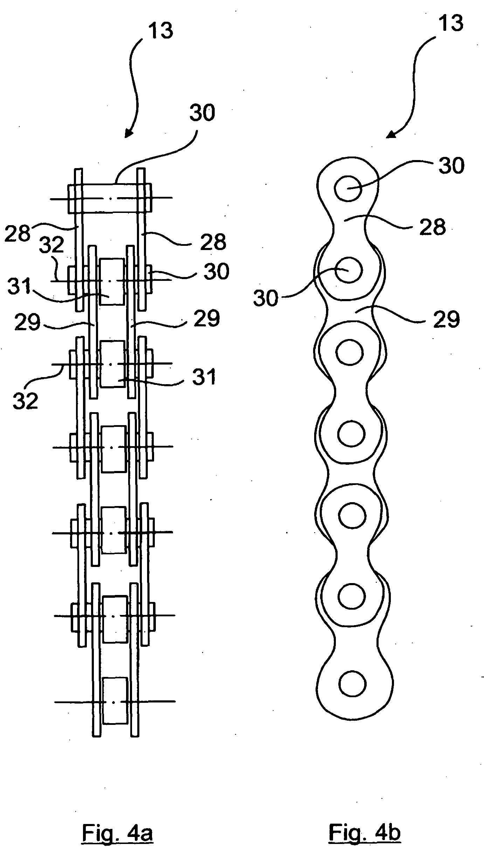

Wie dies insbesondere der Fig. 4a und der Fig. 4b entnommen werden kann, ist die Zugkette hier beispielhaft durch eine eindimensional bewegliche Gelenkkette 13 gebildet, deren Gelenkachsen 32 im Wesentlichen achsparallel zur Drehachse des Kettenrades 15 ausgerichtet sind, wodurch eine zuverlässige Führung des Aggregats 10 sowohl beim Anheben als auch beim Absenken erzielt wird. Insbesondere wird mit einer derartigen Gelenkkette 13 sichergestellt, dass eventuell vorhandene Schwimm- bzw. Schaumschichten 27, die in der Fig. 1 lediglich äußerst schematisch und beispielhaft dargestellt sind, beim Absenken des Aggregats 10 zuverlässig durchdrungen werden können, da die Gelenkkette 13, anders als ein insgesamt biegeschlaffes Seil, keine großen Ausweichbewegungen des Aggregats erlaubt und somit das Durchdringen der Schwimmschicht 27 kontrollierter erfolgen kann.

Zudem ermöglicht eine derartige Gelenkkette 13 als Zugkette im Betrieb des Aggregats 10 eine Stabilisierung desselben, was sich vorteilhaft auf das zum Beispiel Rührergebnis auswirkt.

Wie die Fig. 4a und 4b zeigen, ist die Gelenkkette hier beispielhaft gebildet durch Außenlaschen 28, die über Bolzen 30 miteinander verbunden und voneinander beabstandet sind. An den Bolzen 30 sind Innenlaschen 29 schwenkbar gelagert, wobei zwischen den beiden Innenlaschen auf den Bolzen 30 jeweils Rollen 31 drehbar gelagert sind. Mit einem derartigen beispielhaften Aufbau einer Gelenkkette 13 wird somit eine vorteilhafte Biegesteifigkeit in Richtung der Gelenkachsen 32 sowie in gewissem Maße auch in Kettenlängsrichtung erzielt. Lediglich um die Gelenkachsen 32 herum wird die Gelenkigkeit in gewünschtem Maße bereitgestellt.

Mit einer derartigen Gelenkkette 13 als Zugkette einer Höhenverstelleinrichtung wird somit eine gewünschte Querstabilisierung und Führungsstabilisierung eines Aggregats bei dessen Höhenverstellung ermöglicht, was sich sowohl positiv auf den Absenk- als auch auf den Anhebvorgang auswirkt und zudem auch positive Auswirkungen auf das Rührergebnis mit dem zum Beispiel als Tauchmotorrührgerät ausgebildeten Aggregat 10 zur Folge hat.

Obwohl die vorstehenden Ausführungsbeispiele mit dem Einsatz einer Gelenkkette beschrieben worden sind, kann selbstverständlich anstelle einer derartigen Gelenkkette eine mehrdimensional, insbesondere eine zweidimensional bewegliche Zugkette eingesetzt und verwendet werden, zum Beispiel eine Gliederkette, wie zum Beispiel eine Rundgliederkette.

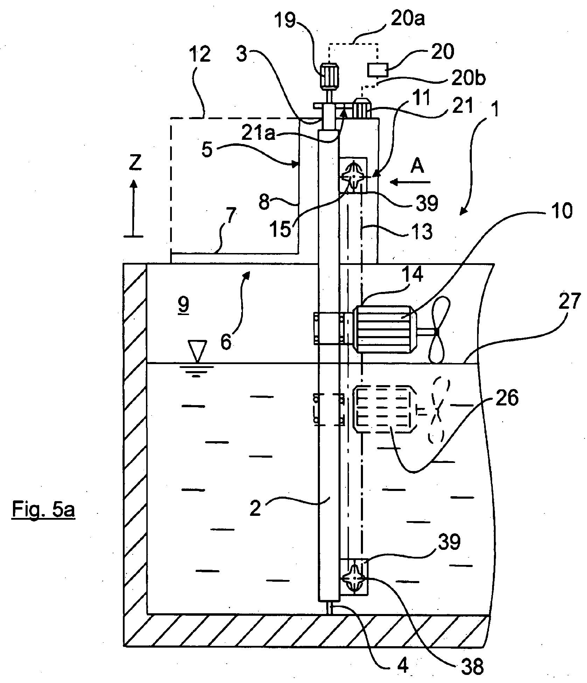

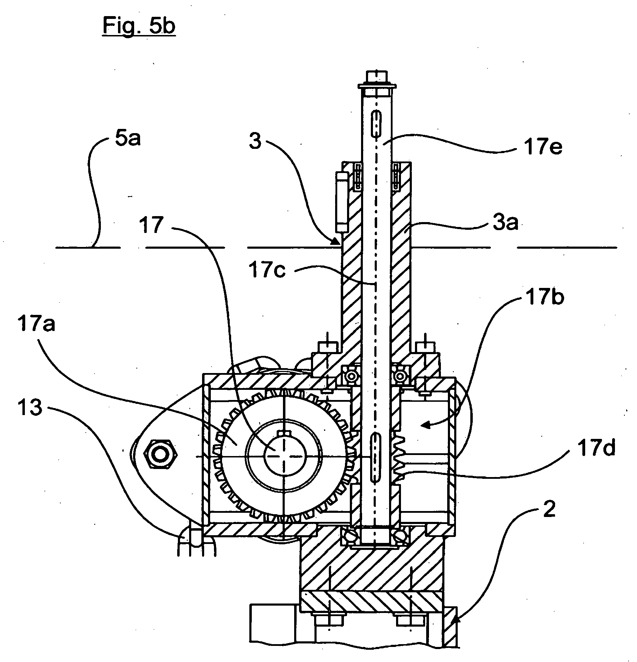

In der Fig. 5a ist zudem eine zur Fig. 1 alternative Ausgestaltung gezeigt, bei der das oben angeordnete Kettenrad 15 Bestandteil einer Antriebswelle 17 ist, auf der ferner ein Antriebsritzel 17a gelagert (Fig. 5b), das mit einem Schneckentrieb 17b zusammenwirkt, der das Antriebsritzel 17a und damit das Kettenrad 15 antreibt. Der Schneckentrieb 17b ist Bestandteil einer Hohlwellenanordnung, bei der in einer das Deckenwand-Drehlager 3 ausbildenden und Bestandteil des Aggregatträgers 2 bildenden Hohlwelle 3a eine Schneckenwelle 17c drehbar gelagert ist, die ein hier mit dem Antriebsritzel 17a direkt und unmittelbar kämmendes Schneckengewinde 17d aufweist (auch die Zwischenschaltung eines oder mehrerer Zahnräder wäre selbstverständlich möglich) und mit einem freien Ende 17e aus dem Fermenterbehälter 1, in der Fig. 5b beispielhaft durch die strichliert eingezeichnete Deckenwand 5a des Serviceschachtes 5 dargestellt, herausgeführt ist. Dort ist ein die Schneckenwelle 17c antreibender Antriebsmotor 19 (Fig. 5a) vorgesehen, der hier lediglich äußerst schematisch und beispielhaft dargestellt ist. Dieser Antriebsmotor 19 kann durch eine Betätigungseinrichtung 20, die ebenfalls außerhalb des Fermenterbeliälters 1 angeordnet ist, betätigt werden, wozu der Antriebsmotor 19 mittels einer hier strichliert dargestellten Signalleitung 20a verbunden ist. Es versteht sich, dass der Antriebsmotor 19 mitsamt Schneckenwelle 17c so ausgebildet ist, dass die Schneckenwelle 17c und damit das Antriebsritzel 17a und das Kettenrad 15 sowohl in die eine als auch in die andere Richtung drehangetrieben werden kann.

Ferner ist außerhalb des Fermenterbehälters 1 auch noch ein weiterer Antriebsmotor 21 angeordnet (Fig.5a), mittels dem über eine hier nicht weiter dargestellte Getriebeanordnung 21 a die mit dem Aggregatträger 2 fest verbundene Hohlwelle 3a und damit der Aggregatträger 2 verschwenkt werden kann. Die Ansteuerung des Antriebsmotors 21 erfolgt ebenfalls über die Betätigungseinrichtung 20 mittels einer Signalleitung 20b. Es sei an dieser Stelle ausdrücklich erwähnt, dass die Verschwenkung des Aggregatträgers 2 alternativ auch manuell, zum Beispiel über einen Schwenkhebel erfolgen kann, was hier aber nicht dargestellt ist. Ebenso könnte auch die Schneckenwelle 17c manuell angetrieben werden, zum Beispiel mit einer Handkurbel, was aber hier nicht dargestellt ist.

## Patentansprüche

1. Biogasanlage,
mit einem Fermenterbehälter (1), in dem ein vertikal ausgerichteter Aggregatträger (2) gelagert ist, wobei ein Aggregat (10) mittels einer Höhenverstelleinrichtung (11) höhenverstellbar an dem Aggregatträger (2) gehaltert ist,
wobei die Höhenverstellvorrichtung (11) durch einen Umschlingungstrieb gebildet ist, dessen Zugmittel (13) um wenigstens zwei in Aggregatträger-Längsrichtung voneinander beabstandete Umlenkelemente (15, 38) geführt ist, und wobei wenigstens eines der Umlenkelemente (15) mittels einer Antriebseinrichtung (19) drehantreibbar ist und wobei das Aggregat (10) dergestalt mit dem Zugmittel (13) gekoppelt ist, dass dieses bei einer Drehbetätigung des Umlenkelementes (15) in eine erste Richtung angehoben und bei einer Drehbetätigung des Umlenkelementes (15) in eine zweite, entgegengesetzte Richtung abgesenkt wird,
**dadurch gekennzeichnet,**
**dass** das Zugmittel (13) von einem Anbindungspunkt (14) am Aggregat (10) ausgehend in Hochachsenrichtung (z) nach oben um ein Umlenkelement (15) und von dort ausgehend nach unten zu wenigstens einem unteren Umlenkelement (38) geführt ist, von wo ausgehend das Zugmittel (13) wieder nach oben zum Aggregat (10) zurückgeführt und dort angebunden ist.

2. Biogasanlage nach Anspruch 1, **dadurch gekennzeichnet, dass** das Zugmittel eine Zugkette (13), vorzugsweise durch eine mehrdimensional bewegliche Kette oder eine lediglich eindimensional bewegliche Gelenkkette ist, wobei bevorzugt vorgesehen ist, dass das wenigstens das drehantreibbare Umlenkelement (15) durch ein Kettenrad gebildet ist, das mit der Zugkette (13) in Eingriff steht.

3. Biogasanlage nach Anspruch 2, **dadurch gekennzeichnet, dass** die Zugkette (13) durch eine Gelenkkette gebildet ist, deren Gelenkachsen (32) im Wesentlichen achsparallel zur Drehachse des Kettenrades (15) ausgerichtet sind, und/oder dass die Gelenkkette durch eine Rollen- oder Buchsen- oder Bolzenkette gebildet ist.

4. Biogasanlage nach einem der vorhergehenden Ansprüche, **dadurch gekennzeichnet, dass** das, vorzugsweise mittelbar oder unmittelbar an dem Aggregatträger (2) angeordnete, untere Umlenkelement (38) drehbar gelagert ist und/oder im Falle einer Zugkette als Zugmittel (13) durch ein Kettenrad gebildet ist.

5. Biogasanlage nach einem der vorhergehenden Ansprüche, **dadurch gekennzeichnet, dass** der Umschlingungstrieb wenigstens ein weiteres Umlenkelement und/oder Spannelement aufweist.

6. Biogasanlage nach einem der vorhergehenden Ansprüche, **dadurch gekennzeichnet, dass** der Umschlingungstrieb im Behälterinnenraum (9) des Fermenterbehälters angeordnet ist und das drehantreibbare Umlenkelement (15) oder eine mit diesem wirkverbundene Antriebseinrichtung (19) mittels einer Betätigungseinrichtung (20) von außerhalb des Fermenterbehälters betätigbar ist.

7. Biogasanlage nach einem der vorhergehenden Ansprüche, **dadurch gekennzeichnet, dass** dem drehantreibbaren Umlenkelement (15) eine Antriebseinrichtung (19), vorzugsweise eine elektrisch, hydraulisch, pneumatisch der manuell betreibbare Antriebseinrichtung (19), zugeordnet ist, mittels der das drehantreibbare Umlenkelement (15) mittelbar oder unmittelbar drehantreibbar ist.

8. Biogasanlage nach Anspruch 7, **dadurch gekennzeichnet, dass** dem das drehantreibbare Umlenkelement (15) Bestandteil einer Antriebswelle (17) ist, die ferner ein Antriebsritzel (17a) aufweist, das mit einem Getriebe (17b) zusammenwirkt, das das Antriebsritzel (17a) und damit das drehantreibbare Umlenkelement (15) antreibt.

9. Biogasanlage nach Anspruch 8, **dadurch gekennzeichnet, dass** das Getriebe (17b) Bestandteil einer Hohlwellenanordnung ist, bei der in einer, bevorzugt ein Deckenwand-Drehlager (3) ausbildenden und/oder Bestandteil des Aggregatträgers (2) bildenden, Hohlwelle (3a) eine Antriebswelle (17c) drehbar gelagert ist, die einen mit dem Antriebsritzel (17a) mittelbar oder unmittelbar kämmenden Eingriffsbereich (17d) aufweist und vorzugsweise mit einem freien Ende (17e) aus dem Fermenterbehälter (1) herausgeführt ist, wobei bevorzugt ein die Antriebswelle (17c) antreibender Antriebsmotor (19) vorgesehen ist, der durch eine Betätigungseinrichtung (20) betätigbar ist.

10. Biogasanlage nach Anspruch 9, **dadurch gekennzeichnet, dass** das Getriebe (17b) ein Schneckentrieb mit einer Schneckenwelle als Antriebswelle (17c) ist, deren Eingriffsbereich (17d) durch ein Schneckengewinde gebildet ist.

11. Biogasanlage nach einem der vorhergehenden Ansprüche, **dadurch gekennzeichnet, dass** die Umlenkelemente (15, 38) des Umschlingungstriebs beabstandet voneinander mittelbar oder unmittelbar am Aggregatträger (2) festgelegt sind.

12. Biogasanlage nach einem der vorhergehenden Ansprüche, **dadurch gekennzeichnet, dass** wenigstens einem der Umlenkelemente (15, 38) wenigstens ein ein- oder mehrteiliges Führungs- und/oder Leitelement (25) so zugeordnet ist, vorzugsweise mit einem definierten Spaltabstand zugeordnet ist, dass damit das Zugmittel (13), insbesondere eine Zugkette, dauerhaft in einer Eingriffsverbindung mit dem zugeordneten Umlenkelement (15, 38), vorzugsweise einem Kettenrad, gehalten ist.

13. Biogasanlage nach Anspruch 12, **dadurch gekennzeichnet, dass** wenigstens ein in Hochachsenrichtung (z) oberes Führungs- und/oder Leitelement (25) so ausgebildet ist, insbesondere so zweigeteilt ausgebildet ist, dass dieses eine Austrittsöffnung (25c) oder ein Austrittspalt für hochgezogene Feststoffe ausbildet.

## Claims

1. Biogas plant,
having a fermenter container (1), in which a vertically oriented unit holder (2) is mounted, a unit (10) being secured on the unit holder (2) in a height-adjustable manner by means of a height adjusting device (11),
the height adjusting apparatus (11) being formed by a wraparound drive, the traction mechanism (13) of which is guided around at least two deflection elements (15, 38) which are spaced apart from one another in the unit carrier longitudinal direction, and it being possible for at least one of the deflection elements (15) to be driven rotationally by means of a drive device (19), and the unit (10) being coupled to the traction mechanism (13) in such a way that it is raised in a first direction during a rotational actuation of the deflection element (15) and is lowered during a rotational actuation of the deflection element (15) in a second, opposite direction,
**characterized**
**in that** the traction mechanism (13) is guided, starting from an attachment point (14) on the unit (10), in the vertical axis direction (z) upwards around a deflection element (15) and, starting from there, downwards to at least one lower deflection element (38), starting from where the traction mechanism (13) is guided upwards again to the unit (10) and is attached there.

2. Biogas plant according to Claim 1, **characterized in that** the traction mechanism is a driving chain (13), preferably a chain which can be moved in multiple dimensions or a link chain which can be moved in merely one dimension, it preferably being provided that the at least one rotationally drivable deflection element (15) is formed by a chain sprocket which is in engagement with the driving chain (13).

3. Biogas plant according to Claim 2, **characterized in that** the driving chain (13) is formed by a link chain, the link axes (32) of which are oriented substantially axially parallel to the rotational axis of the chain sprocket (15), and/or **in that** the link chain is formed by a roller chain or bush chain or pin chain.

4. Biogas plant according to one of the preceding claims, **characterized in that** the lower deflection element (38) which is preferably arranged indirectly or directly on the unit carrier (2) is mounted rotatably and/or is formed by a chain sprocket in the case of a driving chain as traction mechanism (13).

5. Biogas plant according to one of the preceding claims, **characterized in that** the wraparound drive has at least one further deflection element and/or tensioning element.

6. Biogas plant according to one of the preceding claims, **characterized in that** the wraparound drive is arranged in the container interior (9) of the fermenter container, and the rotationally drivable deflection element (15) or a drive device (19) which is operatively connected to the latter can be actuated by means of an actuating device (20) from outside the fermenter container.

7. Biogas plant according to one of the preceding claims, **characterized in that** the rotationally drivable deflection element (15) is assigned a drive device (19), preferably a drive device (19) which can be operated electrically, hydraulically, pneumatically or manually and by means of which the rotationally drivable deflection element (15) can be driven rotationally indirectly or directly.

8. Biogas plant according to Claim 7, **characterized in that** the rotationally drivable deflection element (15) is a constituent part of a drive shaft (17) which, furthermore, has a drive pinion (17a) which interacts with a gear mechanism (17b) which drives the drive pinion (17a) and therefore the rotationally drivable deflection element (15).

9. Biogas plant according to Claim 8, **characterized in that** the gear mechanism (17b) is a constituent part of a hollow shaft arrangement, in which a drive shaft (17c) is mounted rotatably in a hollow shaft (3a) which preferably forms a top wall rotary bearing (3) and/or forms a constituent part of the unit carrier (2), which drive shaft (17c) has an engagement region (17d) which meshes indirectly or directly with the drive pinion (17a), and which drive shaft (17c) is preferably guided with a free end (17e) out of the fermenter container (1), a drive motor (19) which drives the drive shaft (17c) and can be actuated by way of an actuating device (20) preferably being provided.

10. Biogas plant according to Claim 9, **characterized in that** the gear mechanism (17b) is a worm drive with a worm shaft as drive shaft (17c), the engagement region (17d) of which is formed by a worm thread.

11. Biogas plant according to one of the preceding claims, **characterized in that** the deflection elements (15, 38) of the wraparound drive are fixed indirectly or directly on the unit carrier (2) such that they are spaced apart from one another.

12. Biogas plant according to one of the preceding claims, **characterized in that** at least one of the deflection elements (15, 38) is assigned at least one single-part or multiple-part guide and/or guiding element (25), preferably is assigned them/it at a defined gap spacing, in such a way that the traction mechanism (13), in particular a driving chain, is therefore held permanently in an engaged connection with the associated deflection element (15, 38), preferably a chain sprocket.

13. Biogas plant according to Claim 12, **characterized in that** at least one upper (in the vertical axis direction (z)) guide and/or guiding element (25) is configured, in particular is configured so as to be split in two, in such a way that it forms an outlet opening (25c) or an outlet gap for solids which are drawn up.

## Revendications

1. Installation de biogaz :
avec un bac de fermentation (1) dans lequel est disposé un support de groupe (2) orienté verticalement, un groupe (10) étant maintenu de façon réglable en hauteur au niveau du support de groupe (2) au, moyen d'un dispositif de réglage de hauteur (11) ;
le dispositif de réglage de hauteur (11) étant formé d'un entraînement d'enroulement dont le moyen de traction (13) est guidé autour d'au moins deux éléments de déviation (15, 38) espacés l'un par rapport à l'autre dans la direction longitudinale de support de groupe et au moins un des éléments de déviation (15) étant entraîné en rotation à l'aide d'un dispositif d'entraînement (19) et le groupe (10) étant couplé de telle sorte au moyen de traction (13) que celui-ci est soulevé en cas d'actionnement en rotation de l'élément de déviation (15) dans une première direction et est abaissé en cas d'actionnement en rotation de l'élément de déviation (15) dans une deuxième direction opposée ;
**caractérisée en ce que** :
le moyen de traction (13) est guidé vers le haut, dans la direction de l'axe vertical (z), en partant d'un point d'attache (14) situé au niveau du groupe (10), autour d'un élément de déviation (15) et de là vers le bas jusqu'à au moins un élément de déviation (38) inférieur, puis à partir de là, le moyen de traction (13) est de nouveau guidé vers le haut jusqu'au groupe (10) et rattaché à lui à cet endroit.

2. Installation de biogaz selon la revendication 1, **caractérisée en ce que** le moyen de traction est une chaîne de traction (13), de préférence une chaîne mobile dans plusieurs dimensions ou une chaîne articulée mobile dans une seule dimension, sachant que l'on prévoit de façon préférée que l'au moins un élément de déviation (15) entraîné en rotation est formé par un barbotin en prise avec la chaîne de traction (13).

3. Installation de biogaz selon la revendication 2, **caractérisée en ce que** la chaîne de traction (13) est formée par une chaîne articulée dont les axes d'articulation (32) sont orientés pour l'essentiel parallèlement à l'axe par rapport à l'axe de rotation du barbotin (15) et/ou que la chaîne articulée est formée par une chaîne à rouleaux ou à douilles ou à boulons.

4. Installation de biogaz selon l'une quelconque des revendications précédentes, **caractérisée en ce qu'**un élément de déviation (38) inférieur disposé de préférence indirectement ou directement au niveau du support de groupe (2) est disposé de façon à pouvoir tourner et/ou en cas de chaîne de traction servant de moyen de traction (13), est formé par un barbotin.

5. Installation de biogaz selon l'une quelconque des revendications précédentes, **caractérisée en ce que** l'entraînement d'enroulement comporte au moins un élément de déviation et/ou un élément de serrage supplémentaire.

6. Installation de biogaz selon l'une quelconque des revendications précédentes, **caractérisée en ce que** l'entraînement d'enroulement est disposé dans l'espace intérieur de bac (9) du bac de fermentation et que l'élément de déviation (15) entraîné en rotation ou un dispositif d'entraînement (19) relié activement à lui est actionnable depuis l'extérieur du bac de fermentation à l'aide d'un dispositif d'actionnement (20).

7. Installation de biogaz selon l'une quelconque des revendications précédentes, **caractérisée en ce qu'**un dispositif d'entraînement (19), de préférence un dispositif d'entraînement (19) à entraînement électrique, hydraulique, pneumatique ou manuel, est associé à l'élément de déviation (15) entraîné en rotation, l'élément de déviation (15) entraîné en rotation étant indirectement ou directement entraîné en rotation à l'aide dudit dispositif d'entraînement.

8. Installation de biogaz selon la revendication 7, **caractérisée en ce que** l'élément de déviation (15) entraîné en rotation fait partie d'un arbre d'entraînement (17) comportant en outre un pignon d'entraînement (17a) interagissant avec un engrenage (17b) entraînant le pignon d'entraînement (17a) et ainsi l'élément de déviation (15) entraîné en rotation.

9. Installation de biogaz selon la revendication 8, **caractérisée en ce que** l'engrenage (17b) fait partie d'un agencement d'arbre creux dans lequel un arbre d'entraînement (17c) est disposé de façon à pouvoir tourner dans un arbre creux (3a) faisant partie du support de groupe (2) et/ou formant de façon préférée un palier rotatif de paroi supérieure (3), ledit arbre d'entraînement comportant une zone de préhension (17d) s'engrenant indirectement ou directement avec le pignon d'entraînement (17a) et réalisée de préférence avec une extrémité libre (17e) sortant du bac de fermentation (1), un moteur d'entraînement en entrée (19) entraînant l'arbre d'entraînement (17c) étant prévu de façon préférée, ledit moteur pouvant être actionné par un dispositif d'actionnement (20).

10. Installation de biogaz selon la revendication 9, **caractérisée en ce que** l'engrenage (17b) est un entraînement à vis sans fin avec un arbre à vis sans fin servant d'arbre d'entraînement (17c) dont la zone de préhension (17d) est formée par un filet à vis sans fin.

11. Installation de biogaz selon l'une quelconque des revendications précédentes, **caractérisée en ce que** les éléments de déviation (15, 38) de l'entraînement d'enroulement sont fixés indirectement ou directement au support de groupe (2) à une certaine distance l'un de l'autre.

12. Installation de biogaz selon l'une quelconque des revendications précédentes, **caractérisé en ce qu'**au moins un élément de guidage et/ou conducteur (25) réalisé en une ou plusieurs parties est associé à au moins un des éléments de déviation (15, 38) de telle sorte, de préférence avec une distance interstitielle définie, qu'ainsi le moyen de traction (13), notamment une chaîne de traction, est maintenu durablement en liaison avec prise avec l'élément de déviation (15, 38) associé, de préférence un barbotin.

13. Installation de biogaz selon la revendication 12, **caractérisée en ce qu'**au moins un élément de guidage et/ou conducteur (25) placé au-dessus dans la direction de l'axe vertical (z) est réalisé de telle sorte, notamment divisé en deux, que celui-ci forme une ouverture de sortie (25c) ou une fente de sortie pour les matières solides remontées.
